# EUROPEAN PATENT SPECIFICATION

(11) **EP 2 133 113 B1**
(45) Date of publication and mention of the grant of the patent: **26.09.2012**
(21) Application number: 09251355.5
(22) Date of filing: 20.05.2009
(51) Int. Cl.: A61M 16/20, A61M 16/10, F16L 55/10

(54) **Oxygen administration apparatus**
Sauerstoffverabreichungsvorrichtung
Appareil d'administration d'oxygène

(30) Priority: 23.05.2008 GB 0809392; 11.09.2008 GB 0816626
(43) Date of publication of application: 16.12.2009
(73) Proprietor: The BOC Group Limited, The Surrey Research Park Guildford Surrey GU2 7XY (GB)
(72) Inventor: East, Philip Charles, Wolverhampton, West Midlands WV1 2EP (GB)
(74) Representative: Wickham, Michael

(56) References cited:
- GB-A- 2 418 239
- GB-B- 2 417 425
- US-A- 5 927 312

## Description

This invention relates to oxygen administration apparatus.

A conventional apparatus for administering oxygen to a patient comprises a source of oxygen, an oxygen administration device such as a face mask or a nasal cannula and a flexible plastics oxygen conducting line connectible at one end to the source of oxygen and at another end to the administration device.

Many patients, for example, with chronic lung disease now administer oxygen to themselves at home. Oxygen is conventionally supplied as a compressed gas in cylinders. The cylinders are often heavy and patients find them difficult to handle. Moreover, there are considerable logistical problems in ensuring that a patient at home never runs out of oxygen. As a result it is becoming increasingly more commonplace for a so-called oxygen concentrator to be used instead of a cylinder as the source of oxygen. An oxygen concentrator is a device which separates an oxygen-enriched air stream from a flow of atmospheric air. The separation may be effected by an adsorbent or by suitable semi-permeable membranes. If an adsorbent is used, it is typically employed in a plurality of beds. When one bed is adsorbing nitrogen from the air, another bed is being regenerated. In this way a bed with unused adsorptive capacity for nitrogen may be continuously presented to the incoming air, thereby allowing a continuous flow of oxygen-enriched air to be produced. Typically the oxygen concentrator includes a pump or compressor that, in use, maintains a continuous flow of air to the adsorbent or membranes.

Not only do oxygen concentrators avoid the above-mentioned problems associated with compressed oxygen cylinders but they are also safer to use. Nonetheless, it is vital that neither the patient nor anyone else smoke a cigarette or other smokable material in the vicinity of an oxygen administration device irrespective of whether it is supplied with oxygen from a cylinder, from a concentrator or from another source of oxygen. Otherwise, there is an intolerable risk of a serious fire being caused. Patients are therefore given strict instructions not to smoke when administering oxygen or oxygen-enriched air to themselves and not to permit anyone else to smoke in the immediate vicinity of the oxygen administration device. There is, however, the occasional person who is stupid enough to flout these instructions. When this happens there is an immediate risk of a violent conflagration near the patient's face. Moreover, the plastics tubing used to convey the oxygen to the patient will also catch alight and the fire will spread back along the tubing to the concentrator (or oxygen cylinder) itself. There is therefore a long length of burning plastics which may ignite other combustible material, such as soft furnishings, in the patient's living quarters. Indeed, as the tubing burns back towards the concentrator so its combustion is supported by a continuous flow of oxygen-enriched air. Even if a major or fatal fire is avoided, the concentrator can be seriously damaged.

GB-B- 2 417 425 provides a solution to the above described problem by providing an oxygen administration apparatus comprising a source of oxygen, an oxygen administration device, a flexible oxygen conducting line connectible at one end to the source of oxygen and another end to the oxygen administration device, and a safety valve in the line, the safety valve comprising a shuttle, a sealing member carried by the shuttle, means biasing the shuttle in a valve-closing direction, and a fusible stop preventing closure of the valve, whereby fusing of the stop allows the biasing means to close the valve. (Such safety valve is hereinafter referred to as a "firebreak".)

In the event of a fire, the stop fuses causing the safety valve to close immediately and hence the supply of oxygen to cease. Further the safety valve itself may act as a fire break protecting the source of oxygen from the fire.

GB-A-2 418 329 discloses a thermally activatable safety coupling for use in connecting flexible conduits supplying gas. The coupling includes a valve assembly held open against the bias of a negligent means. The coupling also includes a thermally activatable element adopted to release scud valve assembly at a pre-determined temperature, thereby causing the valve assembly to close. The safety coupling can be situated close to a patient's face mask or close to the gas supply.

The oxygen administration apparatus according to GB-B-2 417 425 is now widely used in the United Kingdom to administer oxygen to patients at home.

In the apparatus illustrated in the drawings accompanying GB-B-2 417 425, the firebreak is shown as being located quite near the patient's nasal cannulae (or other oxygen administration device). Indeed, the firebreak may be positioned with one and engaging the nasal cannulae and the other end engaging a single length of flexible tubing suitable for conducting oxygen from a source thereof to the nasal cannulae (or other oxygen administration device). This position of the firebreak is normally effective in limiting the spread of any fire along the tubing to the oxygen source. On rare occasions it has happened that a patient has dropped a cigarette or caused a source of fire to be applied to the tubing between the firebreak and the source of oxygen. In such a situation, although the firebreak is effective to prevent the spread of the fire to the vicinity of the patient's face, it does not prevent further oxygen being fed from the oxygen source to the seat of the fire.

WO-A-2008/075035 discloses arrangements in which a plurality of firebreaks is used in the oxygen line between an oxygen source and nasal cannulae. Each firebreak may have a different configuration at one end from the other. The firebreak at the oxygen source may have a different configuration from the firebreak at the cannulae.

According to the present invention there is provided oxygen administration apparatus comprising a source of oxygen, an oxygen administration device, a length of flexible tubing for conducting oxygen from the source to the administration device, a first connection of a first end of the length of tubing to the oxygen source, the first connection being made through a first firebreak, and a second connection of a second end of the length of tubing to the oxygen administration device, the second connection being made through a second firebreak wherein both firebreaks take the form of a safety valve comprising a shuttle, a sealing member carried by the shuttle, means biasing the shuttle in a valve closing direction, and a fusible stop preventing closure of the valve, whereby fusing of the stop allows the biasing means to close the valve, wherein the firebreaks have first and second connecting ends of different configuration from one another, and wherein the first firebreak has a different configuration from the second firebreak, characterised in that both the first and second firebreaks carry indicia that show the direction in which they are to be connected.

By using two firebreaks, it can be assured that any fire seated along the tubing will not be able to spread along either to the administration device or to the oxygen source, irrespective of the position in the tubing where the fire is seated. The arrangement of the connecting ends reduces the risk of the firebreaks being incorrectly fitted by the patent or by a person assembling the apparatus according to the invention on behalf of the patient.

The first firebreak preferably has a first hollow end with a tapering internal wall adapted to make with a fir tree connector extending from an oxygen concentrator or other form of oxygen. The first firebreak preferably has an elongate hollow second end as the form of a fir tree connector. The length of tubing that is extended to be engaged with the second end of the first firebreak is preferably provided with a trumpet connector which is configured so as to make a fluid-tight engagement with the fir tree connector at the second end of the first firebreak. The interior of the fir tree connector preferably houses an elastomeric or similar O-ring adapted to make a fluid-tight sealing engagement with a spigot member forming part of the trumpet connector.

The second firebreak preferably has an elongate first end in the form of a fir tree connector. The length of tubing that is intended to be engaged with the first end of the second firebreak is preferably provided with a trumpet connector which is configured so as to make a fluid-tight engagement with the fir tree connector at the first end of the second firebreak. The second firebreak preferably has an open, hollow, elongate second end having an external screw thread. The external screw thread is preferably adapted to self tap into a trumpet or similar connector to the oxygen administration device. The second end of the second firebreak is thus of different configuration from the first end thereof. Further the self-tapping nature of the screw-thread facilitates the making of a secure connection to the oxygen administration device. The interior of the elongate screw threaded connector at the second end of the firebreak preferably houses an elastomeric or similar O-ring adapted to make a fluid-tight sealing engagement with a spigot member forming part of the trumpet connector.

Preferably the shuttle has an elongate hollow stem the distal end of which abuts against the fusible stop.

The shuttle is preferable displaceable within a hollow body having a stem complementary to the stem of the shuttle and engageable with the line, the fusible stop engaging the interior of the stem of the body.

The body preferably has an internal surface that acts as a seat for the sealing member when the valve is in its closed position. The body is desirably lightweight but crush-resistant. In order to fulfil both these criteria the body may be formed of aluminium or an aluminium-based alloy.

Preferably there is a restricted passage for the flow of oxygen between the shuttle and the body member.

Preferably the biasing means is a spring. A helical compression spring may be used, but other arrangements are possible. For example, a leaf spring may be used.

There are a number of different arrangements which may be used to seat the helical compression spring. For example, the helical compression spring may be seated on a foot member which engages an insert which in turn engages the proximal end of the hollow body.

The insert preferably has an elongate stem engageable with the length of tubing.

The fusible stop is preferably of a plastics material having a melting point less than that of the material from which the length of tubing is made. The said plastics material may, for example, be an acrylic one or a polyamide.

The oxygen administration device is conveniently a nasal cannula or nasal cannulae.

An oxygen administration apparatus according to the invention will now be described by the way of example with reference to the accompanying drawings, in which:
Figure 1 is a schema of the apparatus;
Figure 2 is a side elevation, partly in section of a firebreak of a kind shown in GB B 2 417 425;
Figure 3 is a schematic sectional side elevation of a firebreak for use as the firebreak 10 in the apparatus shown in Figure 1;
Figure 4 is a schematic sectional side elevation of firebreak for use as the firebreak 12 in the apparatus shown in Figure 1, and
Figure 5 is a schematic view of the connections of the firebreak shown in Figure 4 to a length of tubing and to an oxygen administration device.

The drawings are not to scale.

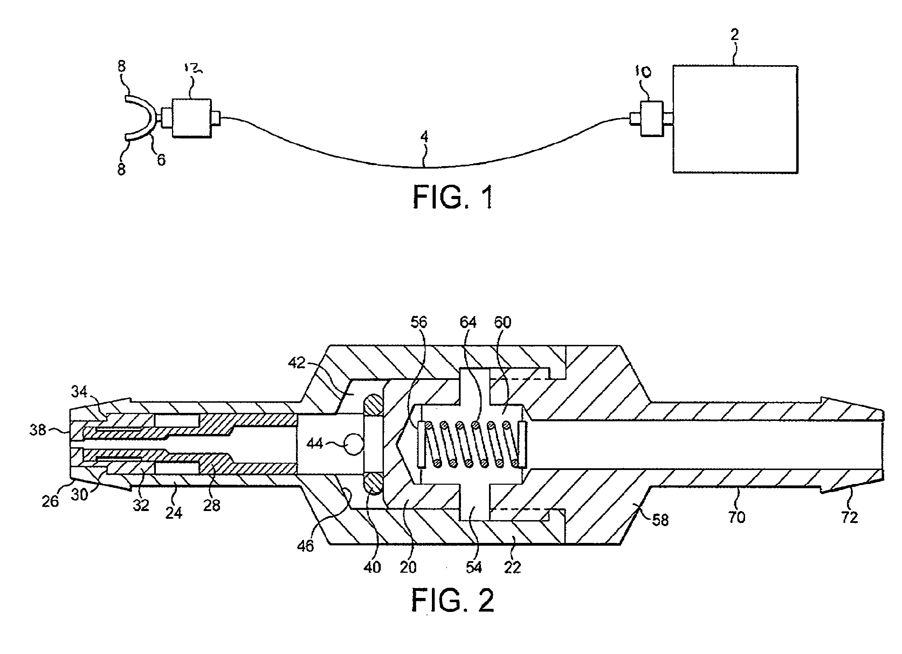

Referring to Figure 1 of the drawings, an oxygen administration apparatus includes an oxygen source 2, which may be an oxygen concentrator or a cylinder container gaseous oxygen under pressure. A length of flexible tubing 4 is attached or connected at one of its ends to the oxygen source 2 and at its other end to an oxygen administration device 6 such as a nasal cannulae having a pair of prongs 8 that the patient can insert in his or her nostrils. The connection of the administration device 6 to the length of tubing 4 is through a firebreak (or safety valve) 12. The connection of the oxygen source to the other end of the length tubing 4 is through another firebreak (or safety valve) 10.

The oxygen concentrator may be of any standard kind that is used for delivering oxygen-enriched air to a patient. Typically, the oxygen concentrator includes an electrically driven pump (not shown) which delivers a continuous flow of air to separations means (not shown) such as a bed or bed adsorbent for semi-permeable membranes. Typically, the operation of the oxygen concentrator is arranged to supply oxygen-enriched air containing about 95% by volume of oxygen. Such oxygen concentrators are well known in the art and need not be described further herein.

The length of tubing 4 is typically formed of polythene although other plastics material may be used instead. Typically the tubing 4 is at least two metres in length.

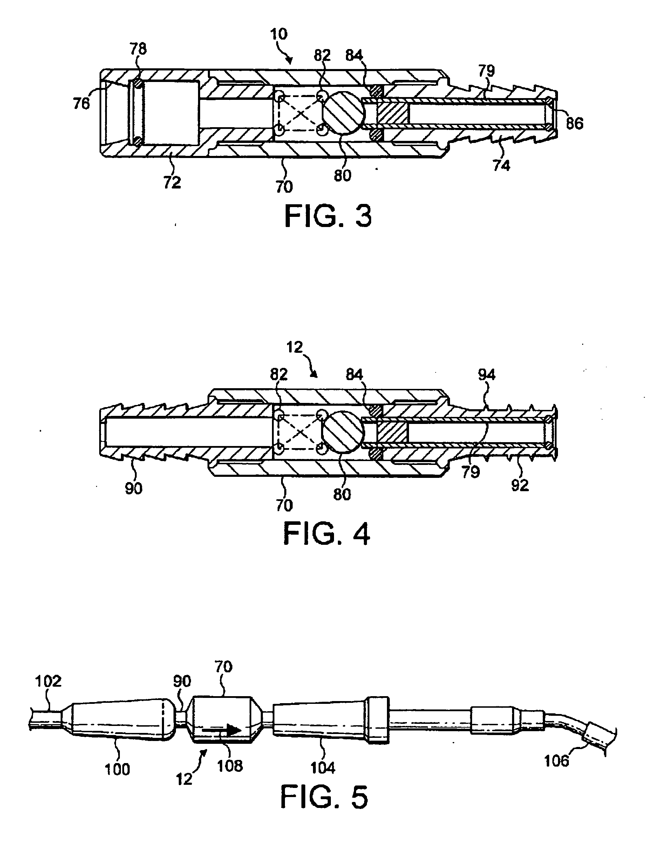

Referring to Figure 2, there is shown a firebreak device according to GB B 2 417 425. This drawing is primarily included so as to illustrate the mechanism by which the firebreak stops the flow of oxygen to a patient in the event of a fire. In the apparatus according to the invention, the first and second firebreaks employ connecting ends of different configuration, as described with reference to Figures 3 and 4. The device shown in Figure 2 comprises a shuttle 20 which is translatable within a hollow body 22. The body 22 has at its distal end an elongate integral stem 24 terminating in an integral nipple 26 to which the necessary connection may be made. The shuttle 20 has at its distal end an integral hollow elongate stem 28 which is complementary to the stem 24 of the body 22.

The interior wall of the nipple 26 is formed with a shoulder 30. A fusible stop 32 is held in frictional engagement between the stem 24 and the stem 28. The stop 32 takes the form of a plastics sleeve 32. The sleeve 32 has a shoulder 34 complementary to the shoulder 30 of the nipple 26. The engagement of the shoulders 30 and 34 prevents the sleeve 32 from being withdrawn through the nipple in normal use of the safety valve. The distal end of the sleeve 32 is formed with a collar 38 which prevents the stem 28 of the shuttle 20 being withdrawn from the valve through the nipple 26 in normal use.

The stem 28 of the shuttle 20 carries at its proximal end a sealing ring 40. As shown in Figure 2, the safety valve 10 is in its open position and, in operation, in this position, oxygen is able to flow from a chamber 42 defined between the shuttle 20 and the body 22 into a port or ports 44 formed in the wall of the stem 28. In its closed position, however, the sealing ring 40 engages internal surface 46 of the body 22 to form a fluid-tight seal. Further, in this closed position, the port 44 is in such a position downstream of the seal that the oxygen from the chamber 42 does not reach it.

The proximal end of the shuttle 20 is formed with a recess 54. Secured to the shuttle 20 in the recess 54 is a head 56. The proximal end of the body 22 receives the distal end of an elongate insert 58. The insert 58 and the body 22 are in engagement with one another such as they cannot readily be separated. The distal end of the insert 58 has a chamber 60. A foot 62 is located within the chamber 60. One end of a compression spring 64 is seated against the foot 62. The other end of the compression spring 64 bears against the head 56. In the normal open position of the safety valve 10 the compression spring 64 is held under compression and therefore acts against the shuttle 20 in a valve - closing direction. However, the stop in the form of the fusible sleeve 32 prevents translation of the sealing ring 40 from the open position in Figure 2 to its closed position in which it abuts against the inner surface 46 of the body 22.

The insert 58 is formed with an integral elongate hollow stem 70 at its proximal end. The end of the stem of 70 is formed as a nipple 72 which can make the necessary connection.

In operation, oxygen in the form of oxygen-enriched air supplied by the oxygen concentrator or other oxygen source flows through the hollow stem 70 of the insert 58. It passes through a restricted passage (not shown) defined between the shuttle 20 and the body 22. Typically, for example, the internal wall of the body 22 may have a hexagonal cross section and the external surface of the shuttle 20 a circular cross-section so as to define gas passages there. The gas flows from these gas passages into the chamber 42. From there the gas flows through the port 44 in the wall of the stem 28 and flows out of the distal end of the valve 10 through the hollow stem 28 of the shuttle 20.

Referring again to Figure 1, in the event of an oxygen fire occurring downstream of the forward firebreak 10, it will cause fusible stop 32 in the form of the sleeve of the firebreak 10 to fuse, thus enabling the compression spring 64 to urge the shuttle 20 forward and carry the sealing ring 40 into valve-closing engagement with the surface 46. Thus, the fire cannot travel along the tubing 4, and the supply of oxygen to the seat of the fire is stopped, the body 22 of the firebreak 10 being made of a material that will not bum under the prevailing conditions.

In the event of an oxygen fire occurring along the length of its tubing 4, the fire travels rapidly backwards towards the oxygen source 2 and the firebreak 12 halts its progress short of the oxygen source 2. The fire causes the fusible stop 32 of the firebreak 12 to fuse, thus enabling the compression spring 64 to urge the shuttle to carry the sealing ring 40 into valve-closing engagement with the surface 46. Thus the flow of oxygen-enriched air (or oxygen) that supports the conflagration is stopped. Further the body 22 acts as physical firebreak , the body being made of material that will not bum under the prevailing conditions. Thus, the fire is prevented from travelling along the oxygen supply line back to the oxygen source 2.

Referring again to Figure 2, the fusible sleeve 32 is made of a plastics material which has a lower melting point than the length of tubing 14. Typically, if the length of tubing 14 is of polythene, the sleeve 30 may be of a suitable low melting point plastics material which may, for example, be a polyamide (such as ACETAL or NYLON).

The shuttle 20 and the body 22 are preferably made of a suitable lightweight material such as aluminium or an aluminium-based alloy. The wall thickness of the body 22 is preferably such as to impart to it a satisfactory degree of crush resistance so that it will not readily be damaged in normal day-to-day use in a domiciliary environment. The engagement of the body 22 and the insert 58 is preferably such as to make it difficult to dismantle the valve without a custom-made tool for disengaging the insert 58 from the body 20.

Referring now to Figure 3, there is shown a device suitable for use as the first firebreak 10 in the apparatus shown in Figure 1. The firebreak 10 has a relatively central hollow body member 70. The body member 70 engages at one end a first connector 72 and at its opposite end a second connector 74. The first connector 72 is configured to be connected to an oxygen concentrator (not shown in Figure 3). The second connector 74 is adapted to be connected to a length of tubing (also not shown) for conducting oxygen to a patient. The first connector 72 is a hollow member open at both ends. It has at its outer end a tapering inner wall 76 defining a generally frusto-conical opening adapted to receive a fir tree connector (not shown) associated with the oxygen concentrator. The material from which the connector 72 is formed has a measure of resilience so as to enable the fir tree connector to be firmly gripped by the surface 76. An O-ring sealing member 78 is located within the connector 72 is also intended to be engaged by the fir tree fitting (not shown). The arrangement is such that different dimensions of fir tree connector may be catered for.

The second connector 74 is itself a fir tree connector, having appropriately shaped projections on its outer surface which enables it to mate with a suitable fitting, for example of the trumpet kind, on the length of tubing to which it is to be connected. It can be seen that the connectors 72 and 74 are of different configuration. This reduces the risk of connecting the device the wrong way around. Further, the body member 70 may have on its external surface an arrow (not shown) indicating the direction of flow of gas through the device. This is another aid to the correct fitting of the firebreak 10.

The inner surface of the connector 74 engages a fusible stop 79 in the form of a tube. The internal end of the tube 79 holds a valve member 80 in position within the body member 70 against the bias of a compression spring 82. In principle, the arrangement of the valve is analogous to that of the device shown in Figure 2. A fire at the patient's end of the apparatus shown in Figure 1 causes the stop 79 to fuse. The compression spring 82 then urges the valve member 80 into a valve-closing engagement with a valve seat 84.

An O-ring 86 is located within the connector 74 at its outer end. The O-ring 86 is engaged, in use, by the spigot of a trumpet connector (not shown in Figure 3) which is engaged with the fir tree connector 74.

The materials of construction of the firebreak 10 shown in Figure 3 may be analogous to those used to make the device shown in Figure 2. The connectors 72 and 74 may be of the same crush-resistant material as the body 70 or may be of a different material. The stop 79 is made of a relatively low melting material.

Referring now to Figure 4, there is shown a device which may be used as the firebreak 12 in the apparatus shown in Figure 1. The firebreak 12 is generally similar in operation to the firebreak 10 shown in Figure 3. The device 12, however, has connectors 90 and 92 of different configuration from the corresponding connectors 72 and 74 of the firebreak 10 as shown in Figure 3. The first connector 90 is adapted to be connected to the opposite end of the length of the tubing to that to which the connector 74 of the firebreak 10, shown in Figure 3 is fitted. The connector 90 is of the fir tree kind and is adapted to be fitted to a trumpet connector on the length of tubing (not shown in Figure 4). The connector 92 is generally similar to the connector 74 of the firebreak 10 shown in Figure 3 save for one important difference. That difference is that the connector 92 does not have an outer surface having a fir tree configuration. Instead, it has a coarse, sharp, screw thread 94. The screw thread is adapted to self tap into a complementary connector (not shown) fitted to an oxygen administration device (not shown). For example, the thread 94 can self tap into the inner surface of a plastics trumpet connector fitted to the oxygen administration device.

Other parts of the firebreak 12 shown in Figure 4 are analogous to the corresponding parts of the firebreak 10 shown in Figure 3 and are therefore indicated by the same reference numerals as used in Figure 3. These parts and their operation will therefore not be described again in relation to Figure 4. The screw threaded configuration of the connector 92 of the firebreak 12 gives it a different configuration from that of the fir tree connector 90 and thus reduces the risk of the firebreak 12 being fitted the wrong way round. This risk can be further reduced by employing on the body 70 of the firebreak 12 an arrow (not shown) indicative of the direction of flow of gas through the device. Another advantage of the screw-threaded configuration of the connector 92 is that enables a particularly secure fitment to an oxygen administration device to be made.

The firebreak 12 may be made of similar materials to the firebreak 10. The fusible stop 78 is made of relatively low melting point material.

Referring now to Figure 5, the firebreak 12 is illustrated in place with its end 90 connected by a trumpet connector 100 to a length of tubing 102 and its connector 92 fitted to another trumpet connector 104 extending from an oxygen administration device 106. It will be seen that the body 70 of the device 12 carries an arrow 108 indicating the direction of oxygen flow.

It will be seen from a comparison of Figure 3 and 4 that the first firebreak 10 has a different configuration from the second firebreak 12 by virtue of their different connecting ends. This is another aid to the correct fitment of the firebreaks.

## Claims

1. Oxygen administration apparatus comprising a source (2) of oxygen, an oxygen administration device (6,106), a length (4,102) of flexible tubing for conducting oxygen from the source (2) to the administration device (6, 106), a first connection of a first end of the length of tubing to the oxygen source (2), the first connection (10) being made through a first firebreak (10), and a second connection (12,100) of a second end of the length of tubing (4,102) to the oxygen administration device (6,106), the second connection being made through a second firebreak (12), wherein both firebreaks (10,12) take the form of a safety valve comprising a shuttle (20), a sealing member (40) carried by the shuttle (20), means (64) biasing the shuttle (20) in a valve closing direction, and a fusible stop (32) preventing closure of the valve, whereby fusing of the stop (32) allows the biasing means to close the valve, wherein both firebreaks (10,12) have first and second connecting ends (90,92) of different configuration from one another, and wherein the first firebreak (10) has a different configuration from the second firebreak (13), **characterised in that** both the first and second firebreaks (10,12) carry indicia (108) that show the direction in which they are to be connected.

2. Oxygen administration apparatus according to Claim 1, wherein the first firebreak (10) has a first hollow open end with a tapering internal wall (76) adapted to mate with a fir tree connector extending from the source of oxygen (2).

3. Oxygen administration apparatus according to claim 1 or claim 2, wherein the first firebreak (10) has an elongate hollow open second exit in the form of a fir tree connector (74).

4. Oxygen administration apparatus according to claim 3, wherein the length of tubing (4) that engages with the second end of the first firebreak (10) does so through a trumpet connector.

5. Oxygen administration apparatus according to any one of preceding claims, wherein the second firebreak (12) has an elongate hollow open first end in the form of a fir tree connector (90).

6. Oxygen administration apparatus according to claim 5, wherein the first end of the second firebreak (12) engages fluid tight with a trumpet connector fitted to the length of tubing.

7. Oxygen administration apparatus according to any one of the preceding claims, wherein the second firebreak (12) has an open, hollow, elongate end (92) having an external screw thread (94).

8. Oxygen administration apparatus according to claim 7, wherein the external screw thread (94) is adapted to self tap into a trumpet connector at the end of the oxygen administration device (6, 106).

9. Oxygen administration apparatus according to claim 1, wherein the shuttle (20) has a hollow stem (20), the distal end of which stem abuts against the fusible stop (32).

10. Oxygen administration apparatus according to claim 9, wherein the shuttle (20) is displaceable within a hollow body (22) having a hollow connecting stem (24) complementary to the stem (28) of the shuttle (20), the fusible stop (32) engaging the interior of the stem (24) of the body (22).

11. Oxygen administration apparatus according to claim 10, in which the body (22) has an internal surface (46) that acts as a seat for the sealing member (40) when the valve is in its closed position.

12. Oxygen administration apparatus according to any one of the preceding claims, wherein the oxygen administration device (6,106) is a nasal cannula.

13. Oxygen administration apparatus according to any one of the preceding claims wherein the source of oxygen (2) is an oxygen concentrator.

## Patentansprüche

1. Sauerstoffverabreichungsvorrichtung, umfassend eine Sauerstoffquelle (2), eine Sauerstoffverabreichungseinrichtung (6, 106), eine Länge (4, 102) eines flexiblen Schlauchs zum Leiten von Sauerstoff von der Quelle (2) zu der Verabreichungseinrichtung (6, 106), eine erste Verbindung eines ersten Endes der Schlauchlänge mit der Sauerstoffquelle (2), wobei die erste Verbindung (10) durch eine erste Feuersperre (10) hergestellt ist, und eine zweite Verbindung (12, 100) eines zweiten Endes der Schlauchlänge (4, 102) mit der Sauerstoffverabreichungseinrichtung (6, 106), wobei die zweite Verbindung durch eine zweite Feuersperre (12) hergestellt ist, wobei beide Feuersperren (10, 12) die Form eines Sicherheitsventils einnehmen, das einen Schieber (20), ein von dem Schieber (20) getragenes Dichtungsglied (40), ein Mittel (64) zum Vorspannen des Schiebers (20) in eine Ventilschließrichtung und einen schmelzbaren schlag (32), der ein Schließen des Ventils verhindert, wobei Schmelzen des schlags (32) dem Vorspannmittel gestattet, das Ventil zu schließen, umfasst, wobei beide Feuersperren (10, 12) ein erstes und ein zweites Verbindungsende (90, 92) mit voneinander verschiedener Konfiguration aufweisen und wobei die erste Feuersperre (10) eine andere Konfiguration aufweist als die zweite Feuersperre (13), **dadurch gekennzeichnet, dass** sowohl die erste als auch die zweite Feuersperre (10, 12) Armierungen (108) aufweisen, die die Richtung zeigen, in der sie zu verbinden sind.

2. Sauerstoffverabreichungsvorrichtung nach Anspruch 1, wobei die erste Feuersperre (10) ein erstes hohles offenes Ende mit einer sich verjüngenden Innenwand (76) aufweist, das zum Zusammenfügen mit einem Tannenbaum-Verbinder, der sich von der Sauerstoffquelle (2) erstreckt, ausgeführt ist.

3. Sauerstoffverabreichungsvorrichtung nach Anspruch 1 oder 2, wobei die erste Feuersperre (10) einen länglichen hohlen offenen zweiten Ausgang in Form eines Tannenbaum-Verbinders (74) aufweist.

4. Sauerstoffverabreichungsvorrichtung nach Anspruch 3, wobei die Schlauchlänge (4), die mit dem zweiten Ende der ersten Feuersperre (10) in Eingriff steht, dies durch einen Trompeten-Verbinder tut.

5. Sauerstoffverabreichungsvorrichtung nach einem der vorhergehenden sprüche, wobei die zweite Feuersperre (12) ein längliches hohles offenes erstes Ende in Form eines Tannenbaum-Verbinders (90) aufweist.

6. Sauerstoffverabreichungsvorrichtung nach Anspruch 5, wobei das erste Ende der zweiten Feuersperre (12) mit einem an der Schlauchlänge angebrachten Trompeten-Verbinder fluiddicht in Eingriff steht.

7. Sauerstoffverabreichungsvorrichtung nach einem der vorhergehenden Ansprüche, wobei die zweite Feuersperre (12) ein offenes hohles längliches Ende (92) mit einem äußeren Schraubgewinde (94) aufweist.

8. Sauerstoffverabreichungsvorrichtung nach Anspruch 7, wobei das äußere Schraubgewinde (94) zum Selbstschneiden in einen Trompeten-Verbinder am Ende der Sauerstoffverabreichungseinrichtung (6, 106) ausgeführt ist.

9. Sauerstoffverabreichungsvorrichtung nach Anspruch 1, wobei der Schieber (20) einen hohlen Schaft (20) aufweist, wobei das distale Ende des Schafts an den schmelzbaren schlag (32) anstößt.

10. Sauerstoffverabreichungsvorrichtung nach Anspruch 9, wobei der Schieber (20) in einem Hohlkörper (22) mit einem hohlen Verbindungsschaft (24), der komplementär zu dem Schaft (28) des Schiebers (20) ist, verschiebbar ist, wobei der schmelzbare schlag (32) das Innere des Schafts (24) des Körpers (22) in Eingriff nimmt.

11. Sauerstoffverabreichungsvorrichtung nach Anspruch 10, wobei der Körper (22) eine Innenfläche (46) aufweist, die als ein Sitz für das Dichtungsglied (40) wirkt, wenn sich das Ventil in seiner geschlossenen Stellung befindet.

12. Sauerstoffverabreichungsvorrichtung nach einem der vorhergehenden sprüche, wobei die Sauerstoffverabreichungseinrichtung (6, 106) eine Nasenkanüle ist.

13. Sauerstoffverabreichungsvorrichtung nach einem der vorhergehenden sprüche, wobei die Sauerstoffquelle (2) ein Sauerstoffkonzentrator ist.

## Revendications

1. Equipement d'administration d'oxygène comprenant une source (2) d'oxygène, un dispositif (6, 106) d'administration oxygène, une longueur (4, 102) de tubulure flexible pour conduire l'oxygène de la source (2) au dispositif (6, 106), un premier branchement d'une première extrémité de la longueur de tubulure à la source (2) d'oxygène, ce premier branchement (10) étant fait par l'intermédiaire d'un premier coupe-feu (10), et un second branchement (12, 100) de la seconde extrémité de la longueur (4, 102) de tubulure au dispositif (6, 106) d'administration d'oxygène, ce second branchement étant fait par l'intermédiaire d'un second coupe-feu (12), dans lequel les deux coupe-feux (10, 12) sont constitués par une valve de sécurité comprenant une navette (20), un élément d'étanchéité (40) porté par la navette (20), un moyen (64) de rappel de la navette (20) dans le sens de la fermeture de la valve et une butée fusible (32) empêchant la fermeture de la valve, moyennant quoi la fusion de la butée (32) permet au moyen de rappel de fermer la valve, dans lequel les deux coupe-feux (10, 12) ont une première et une seconde extrémités de raccordement (90, 92) configurées différemment l'une de l'autre et dans lequel le premier coupe-feu (10) présente une configuration différente de celle du second coupe-feu (13), **caractérisé en ce que** tant le premier que le second des coupe-feux (10, 12) portent des empreintes (108) qui montrent dans quel sens ils doivent être branchés.

2. équipement d'administration d'oxygène selon la revendication 1, dans lequel le premier coupe-feu (10) comporte une première extrémité ouverte creuse avec une paroi interne (76) conique apte à s'accoupler avec un connecteur en sapin s'étendant depuis la source (2) d'oxygène.

3. Équipement d'administration d'oxygène selon la revendication 1 ou 2, dans lequel le premier coupe-feu (10) comporte une seconde sortie ouverte creuse allongée ayant la forme d'un connecteur en sapin (74).

4. Équipement d'administration d'oxygène selon la revendication 3, dans lequel la longueur (4) de tubulure qui s'enclenche sur la seconde extrémité du premier coupe-feu (10) le fait par l'intermédiaire d'un raccord en trompette.

5. équipement d'administration d'oxygène selon l'une quelconque des revendications précédentes, dans lequel le second coupe-feu (12) comporte une première extrémité ouverte creuse allongée ayant la forme d'un connecteur en sapin (90).

6. Équipement d'administration d'oxygène selon la revendication 5, dans lequel la première extrémité du second coupe-feu (12) s'enclenche de façon étanche avec un raccord en trompette monté sur la longueur de tubulure.

7. Equipement d'administration d'oxygène selon l'une quelconque des revendications précédentes, dans lequel le second coupe-feu (12) comporte une extrémité (92) ouverte creuse allongée équipée d'un filetage extérieur (94).

8. Équipement d'administration d'oxygène selon la revendication 7, dans lequel le filetage extérieur (94) est apte à s'autotarauder dans un raccord en trompette à l'extrémité du dispositif (6, 106) d'administration d'oxygène.

9. Équipement d'administration d'oxygène selon la revendication 1, dans lequel la navette (20) comporte une tige creuse (28) dont l'extrémité distale buter contre la butée fusible (32).

10. équipement d'administration d'oxygène selon la revendication 9, dans lequel la navette (20) peut se déplacer à l'intérieur d'un corps creux (22) comportant une tige de liaison creuse (24) complémentaire de la tige (28) de la navette (20), la butée fusible (32) venant au contact de l'intérieur de la tige (24) du corps (22).

11. Équipement d'administration d'oxygène selon la revendication 10, dans lequel le corps (22) comporte une surface interne(46) qui joue le rôle de siège pour l'élément d'étanchéité (40) quand la valve est en position fermée.

12. Équipement d'administration d'oxygène selon l'une quelconque des revendications précédentes, dans lequel le dispositif (6, 106) d'administration d'oxygène est une canule nasale.

13. Équipement d'administration d'oxygène selon l'une quelconque des revendications précédentes, dans lequel la source (2) d'oxygène est un concentrateur d'oxygène.
